# EUROPEAN PATENT APPLICATION

(11) **EP 4 079 685 A1**
(43) Date of publication of application: **26.10.2022**
(21) Application number: 20903373.7
(22) Date of filing: 16.12.2020
(51) Int. Cl.: C01B 25/45, H01M 4/136, H01M 4/58, H01M 10/052, B82Y 30/00

(54) **METHOD FOR PREPARING LITHIUM IRON MANGANESE PHOSPHATE PRECURSOR AND METHOD FOR PREPARING LITHIUM IRON MANGANESE PHOSPHATE**

(30) Priority: 18.12.2019 CN 201911308409
(71) Applicant: Jiangsu Lithitech Co., Ltd., Liyang, Jiangsu 213300 (CN)
(72) Inventor: MA, Yin, Jiangsu 213300 (CN); GONG, Zheng, Jiangsu 213300 (CN); GUO, Yongnan, Jiangsu 213300 (CN); LI, Jiawei, Jiangsu 213300 (CN); LV, Jiale, Jiangsu 213300 (CN)
(74) Representative: Väänänen, Janne Kalervo
(86) International application number: PCT/CN2020/136685
(87) International publication number: WO 2021/121241

(57) **Abstract**

Disclosed are a method for preparing lithium iron manganese phosphate precursor and a method for preparing lithium iron manganese phosphate. The method for preparing lithium iron manganese phosphate precursor comprises the following steps: (1) preparing liquid material A and liquid material B, wherein the liquid material A is a mixed solution of manganese salt and iron salt, and the liquid material B is oxalic acid or phosphoric acid solution; (2) subjecting liquid material A and liquid material B to a co-precipitation reaction in a rotary packed bed (100) to obtain a first slurry; (3) washing and filtering the first slurry to obtain a filter cake; (4) mixing the filter cake with water, adding a carbon source, and stirring until uniform to obtain a second slurry; (5) homogenizing the second slurry; (6) drying the homogenized second slurry, to obtain the lithium iron manganese phosphate precursor. The particle size of the lithium iron manganese phosphate precursor prepared by the method is finer and more uniform than that of a precursor prepared by a traditional method using a reaction kettle, the preparation speed is increased, and the carbon coating is more uniform.

## Description

### TECHNICAL FIELD

The invention belongs to the technical field of chemical crystallization, in particular relates to a method for preparing nanometer-level lithium iron manganese phosphate precursor iron manganese oxalate or iron manganese phosphate by supergravity technology.

### BACKGROUND

With the popularization of various electronic products and electric vehicles, requirements for battery capacity and electric vehicle mileage are constantly increasing. Driven by this trend, high-nickel ternary positive electrode active materials will become an inevitable development direction. Among the ternary positive electrode active materials, the higher the nickel content, the higher the discharge specific capacity after assembling the battery. But everything has two sides, and the high-nickel ternary positive electrode active material is not flawless. Many experts pointed out that the higher the nickel ratio, the worse the thermal stability of the entire positive electrode active material. High temperature instability and poor low temperature performance are the shortcomings of ternary lithium batteries. Thus, safety has become an obstacle that must be overcome first.

Through extensive studies, it is found that blending lithium iron manganese phosphate into the high-nickel ternary positive electrode active material may be one of the best options at this stage. The improvement of high-nickel ternary positive electrode active materials by blending with lithium iron manganese phosphate can be reflected in the following aspects:
The first aspect is safety. The practice of blending high-nickel ternary materials with lithium iron manganese phosphate has been certified by many battery factories. Compared with pure ternary materials, the composite materials prepared by blending ternary materials with 20%-30% lithium iron manganese phosphate increases the thermal decomposition temperature by 5-10% and reduces heat release amount by 40-60%, which proves that the long existed safety problems with the high-nickel ternary materials can be completely solved from the nature of the material;
The second aspect is cost. At present, the cost of ternary materials remains high, especially the price of high-nickel ternary positive electrode active material NCM811 is more than 100% higher than that of lithium iron manganese phosphate materials. Therefore, blending the lithium iron manganese phosphate material into the high-nickel ternary positive electrode can greatly reduce the cost of the material;
The third aspect is battery life. In theory, the cycle life of ternary materials will gradually decrease with the increase of nickel content. At present, the life of high-nickel ternary materials is about 1200-1500 cycles, and it is not realized in full-charge and full-discharge interval, so that the advantages of high energy density of ternary materials are compromised. Lithium iron manganese phosphate is a material that has an olivine structure with high cycle life characteristics. The cycle life after blending ternary materials with lithium iron manganese phosphate will increase by more than 20%-30% depending on the blending ratio.

At present, the application of pure lithium iron manganese phosphate batteries has also been widely recognized. Due to the characteristics of high median voltage plateau, good cycle performance, and good rate performance, pure lithium iron manganese phosphate batteries have great potential in applications of digital 3C batteries and electric two-wheelers.

In order to better match the high-nickel ternary positive electrode active material, the lithium iron manganese phosphate material should meet two requirements. First, the particle size of the lithium iron manganese phosphate should be small, so that it can fill in the voids of the ternary positive electrode active material without affecting the compaction density of the ternary material. Second, the resistivity of the lithium iron manganese phosphate material should be small, which is also the technical barrier of the lithium iron manganese phosphate material.

The strong centrifugal force generated by the rotating packed bed (RPB) makes the fluid involved in the reaction (one of which is liquid) come into flow contact in a porous medium or channel in a supergravity environment that is hundreds to thousands of times larger than the gravitational field of the earth, tearing the liquid to µm or nm-scale liquid films, liquid filaments and droplets, resulting in huge and rapidly renewed phase interfaces. Therefore, the interphase mass transfer and micro-mixing process can be greatly enhanced in the supergravity environment, and the mass transfer coefficient can be increased by 10-1000 times compared with conventional equipment.

The present invention mainly adopts the rotating packed bed equipment to prepare nanometer-level lithium iron manganese phosphate precursor (iron manganese oxalate or iron manganese phosphate). By controlling the reaction process, the particle size and resistivity of the product are controlled during the preparation of the precursor, which lays a solid foundation for the preparation of the final lithium iron manganese phosphate material.

### SUMMARY OF THE INVENTION

### TECHNICAL PROBLEM

One object of the present invention is to prepare a lithium iron manganese phosphate positive active material precursor with stable performance using a supergravity synthesis method and system. Further, this precursor can be calcined to obtain nanometer-level lithium iron manganese phosphate materials with uniform particle size and low resistivity, which overcomes the current technical barriers in the production of lithium iron manganese phosphate.

### TECHNICAL SOLUTION

According to one aspect of the present invention, there is provided a method for preparing lithium iron manganese phosphate precursor, wherein the method comprises the following steps:
(1) preparing a liquid material A and a liquid material B, wherein the liquid material A is a mixed solution of manganese salt and iron salt, and the liquid material B is oxalic acid or phosphoric acid solution,
(2) subjecting the liquid material A and the liquid material B to a co-precipitation reaction in a rotating packed bed to obtain a first slurry,
(3) washing and filtering the first slurry to obtain a filter cake;
(4) mixing the filter cake with water, adding a carbon source, and stirring until uniform to obtain a second slurry;
(5) homogenizing the second slurry;
(6) drying the homogenized second slurry to obtain the lithium iron manganese phosphate precursor.

According to another aspect of the present invention, there is provided a method for preparing lithium iron manganese phosphate, wherein the method comprises:
mixing and drying the lithium iron manganese phosphate precursor obtained in claim 1 with a lithium salt to obtain a mixture; and
calcining the mixture under nitrogen atmosphere to obtain the lithium iron manganese phosphate.

According to yet another aspect of the present invention, there is provided a lithium iron manganese phosphate precursor, the average particle size D50 thereof is 10nm to 1µm, preferably prepared by the above-mentioned method for preparing the lithium iron manganese phosphate precursor.

According to yet another aspect of the present invention, there is provided a lithium iron manganese phosphate, which has a resistivity under 80kg of 10 Ω·cm-500 Ω·cm, preferably prepared by the above-mentioned method for preparing lithium iron manganese phosphate.

According to yet another aspect of the present invention, there is provided a positive electrode comprising the above-mentioned lithium iron manganese phosphate as a positive electrode active material.

According to yet another aspect of the present invention, there is provided a lithium secondary battery comprising the above-mentioned positive electrode.

According to yet another aspect of the present invention, there is provided a battery module comprising the above-mentioned lithium secondary battery as a unit cell.

According to yet another aspect of the present invention, there is provided a battery pack comprising the above-mentioned battery module.

According to yet another aspect of the present invention, there is provided a medium or large device comprising the above-mentioned battery pack as a power source, the medium or large device being selected from the group consisting of an electric tool, an electric vehicle, and a power storage device.

### BENEFICIAL EFFECT

The invention utilizes a rotating packed bed to carry out a supergravity co-precipitation reaction of a mixed solution of manganese salts and iron salts with an oxalic acid solution or a phosphoric acid solution. And then, carbon coating and supergravity homogenization are carried out on the product. After drying, iron manganese oxalate or iron manganese phosphate is obtained as the lithium iron manganese phosphate precursor. Specifically, under the action of the strong centrifugal force generated in the rotating packed bed, the huge shear stress overcomes the surface tension, allowing the liquid to stretch out huge interphase contact interfaces, thereby greatly enhancing the mass transfer process. The liquid entering the rotor is affected by the filler in the rotor, and the strong centrifugal force pushes it to the outer edge of the rotor. During this process, the liquid is dispersed by the filler to form nanometer-level droplets, and nanometer-level materials are co-precipitated.

In addition, the present invention can further utilize the rotating packed bed to carry out the homogenization after carbon coating. Since the solid material can be dispersed in the filler to a great extent during this operation, the specific surface area can be increased, the surface energy can be enhanced, and the homogenization effect can be more uniform, and the homogenization time can be greatly shortened.

Therefore, the method for preparing lithium iron manganese phosphate precursor of the present invention has the following beneficial effects:
1. The particle size of the prepared lithium iron manganese phosphate precursor material is finer and more uniform than that of the precursor material prepared by a traditional method using a reaction kettle.
2. The preparation speed of the method of the present invention is improved by 5-100 times compared with the above-mentioned traditional method.
3. In the obtained lithium iron manganese phosphate precursor material, the carbon coating is more uniform.

In addition, the lithium iron manganese phosphate positive electrode active material obtained by the method for preparing the lithium iron manganese phosphate of the present invention has lower resistivity than that of the lithium iron manganese phosphate positive electrode active material obtained by the traditional method.

### DESCRIPTION OF DRAWINGS

Figure 1 is a schematic diagram of the preparation device and preparation procedure of the lithium iron manganese phosphate precursor.
Figure 2 is a schematic diagram of the preparation device and preparation procedure of the lithium iron manganese phosphate positive electrode active material.
Figure 3 is the first charge-discharge curve of a battery containing the positive electrode active material prepared in Example B1 according to an embodiment of the present invention.
Figure 4 is a cycle performance curve and a charge-discharge efficiency diagram at different rates of a battery containing the positive electrode active material prepared in Example B1 according to another embodiment of the present invention.

### REFERENCES IN THE DRAWINGS

100: Rotating packed bed
101: Liquid material A feeding port
102: Liquid material B feeding port
103: Discharging port

200: Centrifuge
201: Solution feeding port

300: Stirring kettle
301: Filter cake feeding port
302: Carbon source feeding port
303: Stirring kettle discharging port

400: Rotating packed bed
401: Slurry feeding port
402: Homogenized slurry discharging port

500: Spray drying equipment
501: Solution feeding port

600: Mixing system
601: Precursor storage tank
602: Lithium salt storage tank
603: Mixing and stirring kettle

700: Spray dryer
800: Calcining furnace

### DETAILED DESCRIPTIONS

Hereinafter, the present invention will be described in detail with reference to the accompanying drawings. The terms or words used in this specification and claims should not be interpreted as limited to common or dictionary definitions, and should be interpreted as meanings and concepts corresponding to the technical idea of the present invention, on the basis that the inventor can properly define the concept of the terms in the best possible way to describe the principles of the invention.

### 1. Method for preparing lithium iron manganese phosphate precursor

The present invention provides a method for preparing lithium iron manganese phosphate precursor, wherein the method comprises the following steps.

(1.1) Preparing liquid material A and liquid material B, wherein the liquid material A is a mixed solution of manganese salt and iron salt, and the liquid material B is oxalic acid or phosphoric acid solution.

Solid manganese salt and iron salt are mixed in the ratio of 1:9 to 9:1, and then are dissolved in pure water to prepare liquid material A in a concentration of 0.1-3mol. The manganese salt can be one or more of manganese sulfate, manganese acetate, manganese citrate, and manganese chloride, and the iron salt can be one or more of ferrous sulfate, ferrous acetate, and ferrous chloride.

In addition, solid oxalic acid is dissolved in pure water to prepare an oxalic acid solution in a concentration of 0.1-3mol, or phosphoric acid solution is diluted with water to prepare a phosphoric acid solution in a concentration of 0.1-3mol, thereby liquid material B is obtained.

(1.2) Subjecting liquid material A and liquid material B to a co-precipitation reaction in a rotating packed bed to obtain a first slurry containing a precipitate.

The strong centrifugal force generated by the rotating packed bed makes liquid materials A and B come into flow contact in a porous medium or channel in a supergravity environment that is hundreds to thousands times larger than the gravitational field of the earth, tearing them to µm or nm-scale liquid films, liquid filaments and droplets, resulting in huge and rapidly renewed phase interfaces. Therefore, the interphase mass transfer and micro-mixing process of liquid materials A and B are greatly enhanced in the supergravity environment. The mass transfer coefficient of the rotating packed bed can be increased by 10-1000 times compared with conventional equipment.

The rotating packed bed can be selected from a horizontal rotating packed bed and a vertical rotating packed bed.

The co-precipitation reaction means that two or more cations are contained in the solution, and they exist in the solution in a homogeneous phase. After adding a precipitating agent, a uniform precipitate of various components can be obtained after the precipitation reaction. In the present invention, liquid material A provides two cations, manganese ion and iron ion, and liquid material B is a precipitating agent. The advantage of using the co-precipitation method is that the obtained lithium iron manganese phosphate precursor material has a very uniform particle size.

The co-precipitation reaction is carried out in a rotating packed bed, and the obtained product has a homogeneous composition, fine and uniform particle size, and the reaction rate has obvious advantages over other devices. If other reactions are used to prepare the precursors, first, the uniformity of each element of the product cannot be guaranteed; second, the process is relatively complicated, which will lead to a sharp increase in the production cost.

The feeding mode of liquid material A and liquid material B can be one of co-current, counter-current and cross-current.

The feeding speed of liquid material A and liquid material B can be controlled at 10mL-5000mL/min respectively.

The temperature of the co-precipitation reaction can be 20-60°C.

The rotational speed of the rotating packed bed can be 500-3000rpm.

(1.3) Washing and filtering the first slurry is to obtain a filter cake.

After the co-precipitation reaction, the obtained first slurry contains a large amount of sulfate radicals, acetate radicals, citrate radicals or chloride radicals and other substances, so it is necessary to wash and filter the slurry with equipment such as centrifuges, bag filters or suction filtration devices, to remove impurities, and only the precipitate obtained from the co-precipitation reaction, namely the filter cake, is retained.

The equipment used for washing and filtering can be one of centrifugal filter, filter press, bag filter, membrane filter, vacuum suction filter and vacuum filter.

(1.4) Mixing the filter cake with water, adding a carbon source, and stirring until uniform to obtain a second slurry.

The lithium iron manganese phosphate material has poor conductivity due to its own structure. If no carbon source is added, the finally obtained material will not be able to be used as a positive electrode active material. Therefore, carbon coating is required to enhance the conductivity of the positive electrode active material. In the present invention, the conductivity of the subsequently obtained lithium iron manganese phosphate positive electrode active material can be fully enhanced by adding a carbon source.

The carbon source can be an organic carbon source, such as one or more of sucrose, glucose, PVA, PEG, carbon nanotubes, and graphene.

### (1.5) Homogenizing the second slurry.

The second slurry containing the precipitate and the carbon source is homogenized in a homogenizing equipment so that the two substances are uniformly distributed in preparation for the spray drying process. If it is not homogenized, or the homogenization effect is poor, the carbon coating of the obtained material is not uniform. Where the coating layer is too thick, the lithium ion channel will be blocked, affecting the mass transfer process; where the coating layer is too thin, the conductivity will be poor. Therefore, if it is not homogenized, or the homogeneous effect is poor, the electrochemical performance of the positive electrode active material will be significantly affected.

Homogenization can be performed using a rotating packed bed selected from a horizontal rotating packed bed and a vertical rotating packed bed.

(1.6) Drying the homogenized second slurry to obtain the lithium iron manganese phosphate precursor.

The homogenized second slurry is spray dried, and carbon is uniformly coated on the surface of the precipitate while the precipitate is rapidly dried to form a carbon coating layer, thereby obtaining the lithium iron manganese phosphate precursor material.

The inlet temperature of the spray drying equipment can be set to 100-280°C, and the outlet temperature can be set to 50-180°C. The spray drying temperature should be set within an appropriate range, if the temperature is too low, the spraying efficiency will be reduced and the yield will be affected; whereas if the temperature is too high, it will easily cause the oxidation of ferrous and manganese ions in the material, which will affect the performance of the material.

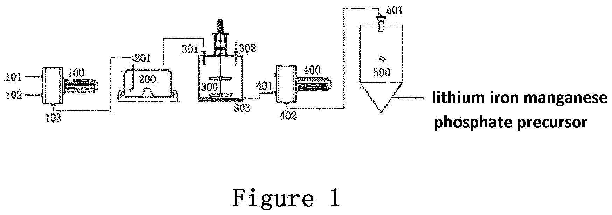

Referring to Figure 1, first, the liquid material A and the liquid material B are pumped into the rotating packed bed 100 through the liquid material A feeding port 101 and the liquid material B feeding port 102 respectively to carry out a co-precipitation reaction, and a first slurry is obtained from the discharging port 103.

Then, the first slurry enters the centrifuge 200 through the solution feeding port 201, and is subjected to centrifugal washing and filtering to obtain a filter cake.

The filter cake is added to the stirring kettle 300 through the filter cake feeding port 301 and mixed with water, a carbon source is added through the carbon source feeding port 302, and the mixture is stirred until uniform to obtain a second slurry.

Then, the second slurry is transported into the rotating packed bed 400 through the stirring kettle discharging port 303 and the slurry feeding port 401, and is homogenized in the rotating packed bed 400.

The homogenized second slurry is output to the spray drying device 500 through the homogenized slurry discharging port 402 and the solution feeding port, and spray drying is performed to obtain the lithium iron manganese phosphate precursor.

### 2. Method for preparing lithium iron manganese phosphate

The present invention provides a method for preparing lithium iron manganese phosphate, which comprises the following steps.

(2.1) Mixing the lithium iron manganese phosphate precursor obtained according to the above with a lithium salt to obtain a mixture.

The lithium salt can be one or more of lithium carbonate, lithium hydroxide, lithium dihydrogen phosphate, lithium citrate, and lithium acetate.

If the lithium salt to be mixed is in solid form, such as solid lithium carbonate, etc., mixing is performed between solid phase and solid phase, and then proceed to the next step; if the lithium salt to be mixed is in liquid form, such as lithium dihydrogen phosphate solution, solid-liquid mixing is performed, and then one step of homogenization and spray drying is performed, the obtained material goes to the next step.

(2.2) Calcining the mixture under nitrogen atmosphere to obtain the lithium iron manganese phosphate.

Calcining can be performed in a calcining furnace, which is one of a roller kiln, a push-plate kiln, a rotary furnace, a box furnace, and a bell jar furnace.

During the calcination process, nitrogen is generally introduced for protection, the calcining temperature can be 300°C to 1000°C, and the calcining time can be 5 to 15 hours. The calcining temperature should be controlled within a suitable range. When the calcining temperature is too low, the crystal form of the material is not easy to form; and when the calcining temperature is too high, the crystal form is too complete, which will make the material relatively hard and not suitable for subsequent processing of the material.

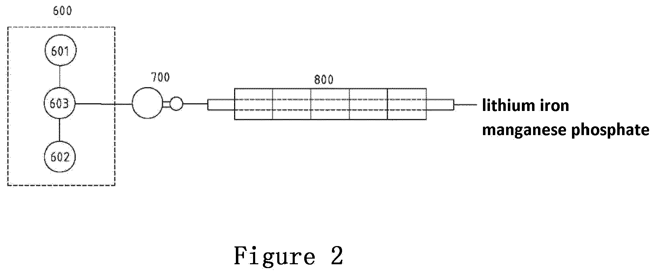

Referring to Figure 2, first, the lithium iron manganese phosphate precursor and the lithium salt are placed in the precursor storage tank 601 and the lithium salt storage tank 602, respectively, and then the lithium iron manganese phosphate precursor and the lithium salt are transported into the mixing and stirring kettle 603 according to a stoichiometric ratio for mixing and stirring. After that, the obtained mixture is transported to the spray dryer 700 for spray drying.

The dried mixture is transferred to the calcining furnace 800 for calcining under a nitrogen atmosphere to obtain the lithium iron manganese phosphate positive electrode active material.

### 3. Lithium iron manganese phosphate precursor and lithium iron manganese phosphate

The present invention provides a lithium iron manganese phosphate precursor, the average particle size D50 thereof is 10nm to 1µm, preferably 10nm to 500nm, more preferably 10nm to 300nm, and still preferably 10nm to 200nm.

The lithium iron manganese phosphate precursor material prepared by the rotating packed bed has a fine particle size, and the minimum particle size can reach about 10-100nm, which lays the foundation for the subsequent preparation of nanometer-level lithium iron manganese phosphate materials. When the lithium iron manganese phosphate material with extremely fine particle size is used in the process of blending with the ternary material, the lithium iron manganese phosphate material can fill and wrap the ternary material, without affecting the compaction density of the material pole piece, thereby improving the volumetric energy density of ternary material batteries. In addition, the lithium iron manganese phosphate fills in the voids of the ternary material. When the ternary material is impacted or sheared by an external force, the lithium iron manganese phosphate material can provide elastic strain force, which improves the safety performance of the ternary material battery.

The present invention also provides a lithium iron manganese phosphate, which has a resistivity under 80kg of 10 Ω·cm-500 Ω·cm, preferably 10 Ω·cm-300 Ω·cm, more preferably 10 Ω·cm-200 Ω·cm, still preferably 10 Ω·cm to 100 Ω·cm. The lithium iron manganese phosphate can be used as a positive electrode active material. The resistivity of the lithium iron manganese phosphate material prepared by the invention is extremely low under 80kg, and the minimum can reach about 10 Ω·cm. When the lithium iron manganese phosphate with low resistivity is mixed with the ternary positive electrode material, it not only will not affect the overall resistivity of the material, but also can build more lithium ion channels for the intercalation and deintercalation of lithium ions, which greatly improves the electrochemical properties of blended materials.

The present invention also provides a positive electrode for lithium secondary battery, and the positive electrode comprises the lithium iron manganese phosphate according to the present invention as a positive electrode active material. The positive electrode for lithium secondary battery includes a positive electrode current collector and a positive electrode active material layer formed on the positive electrode current collector, and the positive electrode active material layer comprises the positive electrode active material according to the present invention.

The present invention also provides a lithium secondary battery comprising the above-mentioned positive electrode.

The present invention also provides a battery module comprising the lithium secondary battery as a unit cell.

The lithium secondary battery according to the present invention can be used as a unit cell of a battery module, and the battery module can be applied to a battery pack. The battery pack can be used as a power source for at least one of the following medium and large equipment: electric tools; electric vehicles, including pure electric vehicles (EV), hybrid electric vehicles (HEV), and plug-in hybrid electric vehicles (PHEV); or power storage devices.

### PREFERRED EMBODIMENT

Hereinafter, the present invention will be described in detail with reference to examples to specifically describe the present invention. However, the examples of the present invention may be modified into various other forms and the scope of the present invention should not be construed as being limited to the examples described below. The examples of the present invention are provided to more fully describe the present invention to those of ordinary skill in the art.

The experimental methods in the following examples, if no specific conditions are indicated, are usually performed under conventional conditions in the field or conditions suggested by the manufacturer; the raw materials and equipment used, unless otherwise specified, can be obtained from commercial channels such as conventional markets.

### Example A1

The lithium iron manganese phosphate precursor was prepared by the following procedure.
(1) Preparing a mixed solution of ferrous sulfate and manganese sulfate at 2 mol/L (wherein the molar ratio of iron to manganese was 5:5), and an oxalic acid solution at 2 mol/L.
(2) Pumping the mixed solution and the oxalic acid solution in a co-current manner into a rotating packed bed (vertical rotating packed bed, Hangzhou Ke-Li Chemical Equipment Co., Ltd., BZ650-3P) at a feeding rate of 1:1 (volume ratio) for a co-precipitation reaction, wherein the reaction temperature in the rotating packed bed was 25°C, the feeding rates of the two raw materials were controlled at 100mL/min, and the rotational speed of the rotating packed bed was 2000rpm. A first slurry of iron manganese oxalate was obtained.
(3) Centrifuging and washing the first slurry of iron manganese oxalate by a centrifuge (Jiangsu Shengli Centrifuge Manufacturing Co., Ltd., PSD/SD1000) to obtain an iron manganese oxalate filter cake.
(4) Mixing the obtained iron manganese oxalate filter cake with pure water, feeding into the stirring kettle together, and then adding glucose as an organic carbon source to make the solid content 20%. Turning on the stirring kettle, controlling the stirring speed at 200 rpm, and stirring for 2 h to obtain a second slurry.
(5) Pumping the second slurry into a homogenizer (Nantong Claire, JMA22 jet-flow dispersing emulsification homogenizer) for homogenization.
(6) Spray-drying the homogenized second slurry through a spray dryer, the inlet temperature of the spray dryer was 200°C, and the outlet temperature was 120°C, to obtain solid iron manganese oxalate, which was the lithium iron manganese phosphate precursor.

### Example A2

The same procedure as in Example A1 was adopted, except that in step (1), oxalic acid solution at 2 mol/L was replaced with phosphoric acid solution at 2 mol/L. Finally, solid iron manganese phosphate was obtained, which was the lithium iron manganese phosphate precursor.

### Example A3

The same procedure as in Example A1 was adopted, except that in step (5), the homogenizer was replaced with a rotating packed bed (vertical rotating packed bed, Hangzhou Ke-Li Chemical Equipment Co., Ltd., BZ650-3P). Finally, solid iron manganese oxalate was obtained, which was the lithium iron manganese phosphate precursor.

### Example A4

The same procedure as in Example A2 was adopted, except that in step (5), the homogenizer was replaced with a rotating packed bed (vertical rotating packed bed, Hangzhou Ke-Li Chemical Equipment Co., Ltd., BZ650-3P). Finally, solid iron manganese phosphate was obtained, which was the lithium iron manganese phosphate precursor.

### Comparative Example A1

The same procedure as in Example A1 was adopted, except that in step (2), the rotating packed bed was replaced with a co-precipitation reactor. Finally, iron manganese oxalate was obtained, which was the lithium iron manganese phosphate precursor.

### Comparative Example A2

The same procedure as in Example A2 was adopted, except that in step (2), the rotating packed bed was replaced with a co-precipitation reactor. Finally, solid iron manganese phosphate was obtained, which was the lithium iron manganese phosphate precursor.

### Experimental Example 1 Particle Size Test

A laser particle size analyzer (OMECLS-POP) was used to test the particle size D10, D50, D90 of the product. First, 2g of the powder to be tested was taken and put in a beaker, then 100mL of pure water and 2mL of dispersing agent was added and put into an ultrasonic oscillator for ultrasonic dispersion for 5 minutes, and then poured into a laser particle size analyzer, where the refractive index of the medium was set to 1.33, and the refractive index of the material was set to 1.741, and D10, D50, and D90 parameters were tested. Three sets of data were tested in parallel for each sample and averaged, and the results are shown in Table 1 below.

**Table 1**

| Sample | D10 | D50 | D90 |
|---|---|---|---|
| Example A1 | 121nm | 173 nm | 276 nm |
| Example A2 | 155 nm | 206 nm | 326 nm |
| Example A3 | 133nm | 201nm | 312nm |
| Example A4 | 148nm | 199nm | 284nm |
| Comparative Example A1 | 3.65µm | 10.12µm | 18.37µm |
| Comparative Example A2 | 2.47µm | 8.43µm | 15.51µm |

As can be seen from Table 1, in Examples A1, A2, A3 and A4, lithium manganese iron phosphate precursors were prepared by supergravity technology, and the particle size D50 of the two precursors were both nanometer-level, and the particle size distribution was narrow, showing that the particle size distribution was very uniform.

In Comparative Example 1 and Comparative Example 2, co-precipitation reactor was used to prepare the lithium iron manganese phosphate precursor material, and the particle size D50 of the prepared precursor was 10.12µm and 8.43µm, which were larger, and the particle size distribution range was wider, showing that the particle size was not uniform.

### Example B1

The iron manganese oxalate in Example A1 was mixed with lithium dihydrogen phosphate in a stoichiometric ratio, and after being spray-dried, it was calcined at 700°C for 15 hours in a rotary furnace under nitrogen protection to obtain a lithium iron manganese phosphate material.

### Example B2

The iron manganese phosphate in Example A2 was mixed with lithium carbonate in a stoichiometric ratio, and then calcined at 700°C for 15 hours in a rotary furnace under nitrogen protection to obtain a lithium iron manganese phosphate material.

### Example B3

The iron manganese oxalate in Example A3 was mixed with lithium dihydrogen phosphate in a stoichiometric ratio, and after being spray dried, it was calcined at 700 °C for 15 hours in a rotary furnace under nitrogen protection to obtain a lithium iron manganese phosphate positive electrode material.

### Example B4

The iron manganese phosphate in Example A4 was mixed with lithium carbonate in a stoichiometric ratio, and then calcined at 700°C for 15 hours in a rotary furnace under nitrogen protection to obtain a lithium iron manganese phosphate material.

### Comparative Example B 1

The iron manganese oxalate in Comparative Example A1 was mixed with lithium dihydrogen phosphate in a stoichiometric ratio, and after being spray dried, it was calcined at 700°C for 15 hours in a rotary furnace under nitrogen protection to obtain a lithium iron manganese phosphate material.

### Comparative Example B2

The iron manganese phosphate in Comparative Example A2 was mixed with lithium carbonate in a stoichiometric ratio, and then calcined at 700°C for 15 hours in a rotary furnace under nitrogen protection to obtain a lithium iron manganese phosphate material.

### Experimental Example 2 Resistivity Test

A resistivity meter (Lattice Electronics SZT-D) was used to test the resistivity of the product. First, take a certain amount of powder to be tested and put it in the silo of the resistivity meter until the silo was filled, adjust the pressure to 40.0kg to test its resistivity under 40kg, and then continue to increase the pressure to 80kg to test its resistivity under 80kg. Three sets of data were tested in parallel for each sample and averaged, and the results are shown in Table 2 below.

**Table 2**

| Sample | Resistivity (40kg) | Resistivity (80kg) |
|---|---|---|
| Example B1 | 155.2Ω·cm | 123.8Ω·cm |
| Example B2 | 183.5 Ω·cm | 144.7Ω·cm |
| Example B3 | 46.9 Ω·cm | 31.9 Ω·cm |
| Example B4 | 97.7 Ω·cm | 69.0 Ω·cm |
| Comparative Example B1 | 698.3Ω·cm | 572.1Ω·cm |
| Comparative Example B2 | 798.4Ω·cm | 688.8Ω·cm |

As can be seen from Table 2, in Examples B1 and B2, the lithium iron manganese phosphate materials prepared using the rotating packed bed are fine and uniform in particle size, and have a huge specific surface area, which makes carbon coating easier and more uniform. Therefore, the resistivity of the obtained iron manganese phosphate positive electrode material is significantly lower than that of the lithium iron manganese phosphate material produced by other processes.

In Examples B3 and B4, nanometer-level precursors were prepared using the rotating packed bed, and homogenization before carbon coating was carried out using the rotating packed bed. The lithium iron manganese phosphate material produced by this process can not only make the particle size fine and uniform, but also make carbon uniformly coated on the surface of the material. Therefore, the resistivity of the lithium iron manganese phosphate material prepared in Examples B3 and B4 is even lower.

In Comparative Examples B1 and B2, the precursor materials were prepared using the co-precipitation reactor, and a common homogenizer was used for homogenization before carbon coating. The particle size D50 of the obtained precursor materials is micron level, the particle size is large, and the surface energy is small, the uniformity after carbon coating is poor. Thick carbon coating is easy to affect the lithium ion conduction, and thin coating is easy to affect the electron conduction, which ultimately affects the overall electrochemical performance of the material. The resistivity is relatively high, and the resistivity under 80kg is more than 500Ω·cm.

### Experimental example 3 Electrochemical test

Electrochemical tests were performed on the lithium iron manganese phosphate material (positive electrode active material) prepared in Example B1. The electrochemical tests were performed in the same way as on a CR2032 coin-type half-cell.

The CR2032 coin-type half-cell was prepared as follows.

First, a positive electrode slurry was prepared. Specifically, prepare 10 parts by weight of PVDF (which is dissolved in N-methyl-2-pyrrolidone (NMP), wherein PVDF:NMP=3:100 (by weight)), stir until uniform, and then add 10 parts by weight of Super P and 80 parts by weight of the positive electrode active material, stir all until uniform to obtain a positive electrode slurry.

Then, the positive electrode slurry was uniformly coated on the aluminum foil, placed in a vacuum oven, and dried at 120°C for 10 hours, so that all NMP in the positive electrode slurry was volatilized to obtain a positive electrode sheet.

Then, the positive electrode sheet was cut into 15mm diameter discs, put into a tablet press, and pressed at a pressure of IMPa for 5s.

Then, the pressed positive electrode sheet was put into a glove box for battery assembly, and the glove box was protected by an argon atmosphere. Wherein, pure lithium sheet was used as a negative electrode sheet; polyethylene was used as a separator; and the solution obtained through dissolving 1mol/L lithium hexafluorophosphate in a mixed solvent of ethylene carbonate and diethyl carbonate having a molar ratio of 1:1 was used as electrolyte solution. The positive electrode sheet, the separator, and the negative electrode sheet are pressed together to prepare an electrode assembly, which is then placed in a battery case. After that, the electrolyte was injected into the case to produce a CR2032 coin-type half-cell.

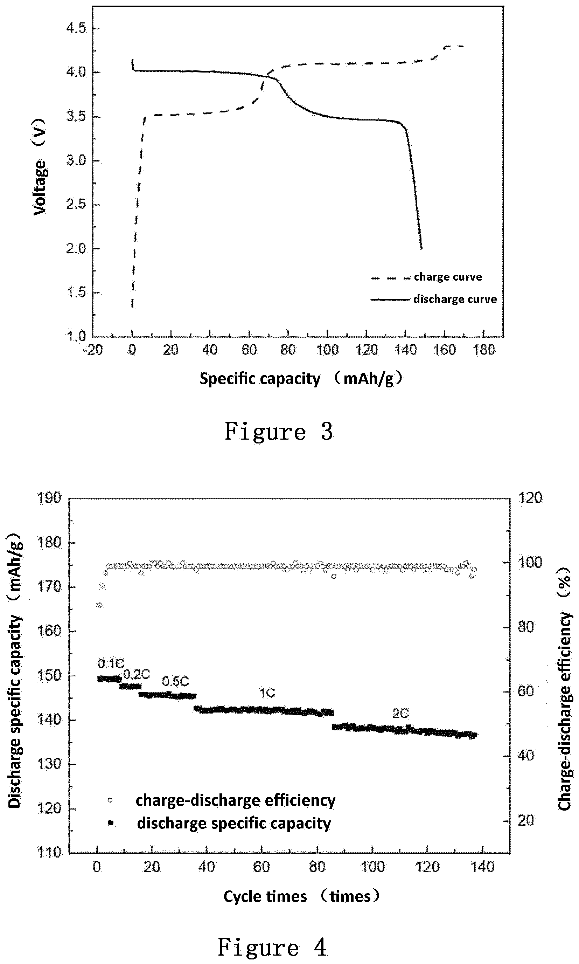

The electrical performance test of the battery includes cycle performance test and rate performance test. The cycle performance test and the rate performance test are both measured in a battery tester (Neware, CT-3008) at different charge and discharge rates in the voltage range of 2.5V-4.3V through different times of full-charge and full-discharge. Among them, the test is performed for 8 times at a rate of 0.1C, 8 times at a rate of 0.2C, 20 times at a rate of 0.5C, 50 times at a rate of 1C, and 50 times at a rate of 2C. The results of the measurements are shown in Figures 3 and 4.

Figure 3 is the first charge-discharge curve of a battery containing the lithium iron manganese phosphate material (positive electrode active material) prepared in Example B1.

As can be seen from Figure 3, the battery containing the positive electrode active material prepared in Example B1 has two voltage plateaus during the charging and discharging process, which are the voltage plateaus of manganese and iron respectively. Both voltage plateaus are relatively stable, and voltage value of the manganese voltage plateau is relatively high, so that the median voltage plateau is relatively high.

In addition, as can be seen from Figure 3, the specific capacity of the first charge of the battery can reach 165mAh/h, the specific capacity of the first discharge is 150mAh/g, and the first charge-discharge efficiency can reach 90.9%, which is significantly higher than that of positive electrodes containing other lithium iron manganese phosphate.

Figure 4 is a cycle performance curve and a charge-discharge efficiency diagram at different rates of a battery containing the lithium iron manganese phosphate material (positive electrode active material) prepared in Example B1.

As can be seen from Figure 4, the discharge specific capacity can be maintained at about 150mAh/g at a rate of 0.1C, the discharge specific capacity can be maintained above 140mAh/g at a rate of 1C, and the discharge specific capacity can reach nearly 140mAh/g at a rate of 2C. It can be seen that the rate performance of the battery containing the lithium iron manganese phosphate material of the present invention is better.

The above are only the preferred embodiments of the present invention. It should be pointed out that for those of ordinary skill in the art, without departing from the concept of the present invention, certain improvements and modifications can also be made, and these improvements and modifications should also be regarded as within the protection scope of the present invention.

## Claims

1. A method for preparing lithium iron manganese phosphate precursor, wherein the method comprises the following steps:
(1) preparing a liquid material A and a liquid material B, wherein the liquid material A is a mixed solution of manganese salt and iron salt, and the liquid material B is oxalic acid or phosphoric acid solution;
(2) subjecting the liquid material A and the liquid material B to a co-precipitation reaction in a rotating packed bed to obtain a first slurry;
(3) washing and filtering the first slurry to obtain a filter cake;
(4) mixing the filter cake with water, adding a carbon source, and stirring until uniform to obtain a second slurry;
(5) homogenizing the second slurry;
(6) drying the homogenized second slurry to obtain the lithium iron manganese phosphate precursor.

2. The method according to claim 1, wherein in step (1), the manganese salt is one or more of manganese sulfate, manganese acetate, manganese citrate, and manganese chloride, and
the iron salt is one or more of ferrous sulfate, ferrous acetate, and ferrous chloride.

3. The method according to claim 1, wherein in step (1), the concentration of the liquid material A is 0.1-3mol/L, and the concentration of the liquid material B is 0.1-3mol/L.

4. The method according to claim 1, wherein in step (2), the rotating packed bed is selected from a horizontal rotating packed bed and a vertical rotating packed bed.

5. The method according to claim 1, wherein in step (2), the feeding mode of the liquid material A and the liquid material B is one of co-current, counter-current and cross-current, and
the feeding speed of the liquid material A and the liquid material B is controlled at 10mL-5000mL/min, respectively.

6. The method according to claim 1, wherein in step (2), the temperature of co-precipitation reaction is 20-80°C, and the rotational speed of the rotating packed bed is 500-3000rpm.

7. The method according to claim 1, wherein in step (3), the equipment for washing and filtering is one of centrifugal filter, filter press, bag filter, membrane filter, vacuum suction filter and vacuum filter.

8. The method according to claim 1, wherein in step (4), the carbon source is one or more of sucrose, glucose, PVA, PEG, carbon nanotubes, and graphene.

9. The method according to claim 1, wherein in step (5), homogenizing is performed using a rotating packed bed selected from a horizontal rotating packed bed and a vertical rotating packed bed.

10. The method according to claim 1, wherein in step (6), drying is carried out using spray drying equipment, the inlet temperature of the spray drying equipment is set to 100-280°C, and the outlet temperature is set to 50-180°C.

11. A method for preparing lithium iron manganese phosphate, wherein the method comprises:
mixing and drying the lithium iron manganese phosphate precursor obtained in claim 1 with a lithium salt to obtain a mixture; and
calcining the mixture under nitrogen atmosphere to obtain the lithium iron manganese phosphate.

12. The method according to claim 11, wherein the lithium salt is one or more of lithium carbonate, lithium hydroxide, lithium dihydrogen phosphate, lithium citrate, and lithium acetate;
the drying equipment is a spray dryer, preferably, the inlet temperature of the spray dryer is 100-280°C, and the outlet temperature is 50-180°C;
calcining is carried out in a calcining furnace, which is one of a roller kiln, a push-plate kiln, a rotary furnace, a box furnace, and a bell jar furnace, preferably, the calcining temperature is 300°C-1000°C, and the calcining time is 5 to 15 hours.

13. A lithium iron manganese phosphate precursor having an average particle size D50 of 10nm to 1µm, preferably prepared by the method according to any one of claims 1-10.

14. A lithium iron manganese phosphate having a resistivity under 80kg of 10 Ω·cm-500 Ω·cm, preferably prepared by the method of any one of claims 11-12.

15. A positive electrode comprising the lithium iron manganese phosphate according to claim 14 as a positive electrode active material.

16. A lithium secondary battery comprising the positive electrode according to claim 15.

17. A battery module comprising the lithium secondary battery according to claim 16 as a unit cell.

18. A battery pack comprising the battery module according to claim 17.

19. A medium or large device comprising the battery pack according to claim 18 as a power source, the medium or large device being selected from the group consisting of an electric tool, an electric vehicle, and a power storage device.
